(19) **Europäisches Patentamt European Patent Office Office européen des brevets**

(11) **EP 4 613 216 A1**

(12) **EUROPEAN PATENT APPLICATION**
published in accordance with Art. 153(4) EPC

(43) Date of publication:
**10.09.2025  Bulletin 2025/37**

(21) Application number: **23911708.8**

(22) Date of filing: **13.12.2023**

(51) International Patent Classification (IPC):
***A61B 17/29*** *(2006.01)*

(52) Cooperative Patent Classification (CPC):
**A61B 17/29**

(86) International application number:
**PCT/JP2023/044577**

(87) International publication number:
**WO 2024/142927 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **27.12.2022   JP 2022209660**

(71) Applicant: **KAWASAKI JUKOGYO KABUSHIKI KAISHA**
**Kobe-shi, Hyogo 650-8670 (JP)**

(72) Inventors:
• **MATSUURA, Takeshi**
  **Hyogo 650-8670 (JP)**
• **AIBE, Toru**
  **Hyogo 650-8670 (JP)**
• **SATO, Eiji**
  **Hyogo 650-8670 (JP)**
• **ORYU, Tamami**
  **Hyogo 650-8670 (JP)**

(74) Representative: **Hoffmann Eitle**
**Patent- und Rechtsanwälte PartmbB**
**Arabellastraße 30**
**81925 München (DE)**

(54) **SURGICAL INSTRUMENT**

(57)     A surgical instrument (100) includes a rod mover (50) to allow a proximal end of a rod (40) to be fixed thereto, a drive (61) to reciprocate the rod mover (50) in an axial direction of a shaft (10), and a tube (81) between the rod mover (50) and the shaft (40), and the tube (81) is inserted into a hole (51) of the rod mover (50).

*FIG.4*

## Description

Technical Field

The present disclosure relates to a surgical instrument.

Background Art

Conventionally, a surgical instrument including a bent portion is disclosed. For example, Japanese Patent Laid-Open No. 2014-038075 discloses a forceps manipulator including a bendable portion and a gripper, and a spring is used in the bendable portion. Four holes are formed in a wall surface of the spring. Rods are inserted into the holes. The rods are superelastic rods. Distal ends of the rods are fixed to the spring. Proximal ends of the four rods are each connected to a pneumatic cylinder. The bendable portion is bent by the rods being pushed by the pneumatic cylinder and the rods being pulled by the pneumatic cylinder. In addition, a shaft is connected to the proximal end side of the bendable portion. The rods are disposed in the shaft of the forceps manipulator. In the shaft, the rods are inserted into guide pipes.

Prior Art

Patent Document

Patent Document 1: Japanese Patent Laid-Open No. 2014-038075

Summary of the Invention

In the forceps manipulator disclosed in Japanese Patent Laid-Open No. 2014-038075, it is necessary to increase the stroke of the pneumatic cylinder to increase the amount of pushing the rods when the bending angle of the bendable portion is increased. In such a case, the buckling load is reduced, and the rods buckle when the same are pushed even slightly. In Japanese Patent Laid-Open No. 2014-038075, the rods are exposed between the shaft and the pneumatic cylinder and are not covered with the guide pipes, and thus the rods may conceivably buckle between the shaft and the pneumatic cylinder when a force applied to the rods increases. Thus, the bending angle of the bendable portion cannot be increased.

The present disclosure is intended to solve the above problem. The present disclosure aims to provide a surgical instrument that enables the bending angle of a bendable portion to be increased.

A surgical instrument according to an aspect of the present disclosure includes a shaft, a bendable portion connected to a distal end of the shaft, an end effector at a distal end of the bendable portion, a rod inserted into the shaft and the bendable portion and including a distal end fixed to an end effector side of the bendable portion, a rod mover to allow a proximal end of the rod to be fixed thereto, a drive to reciprocate the rod mover in an axial direction of the shaft, and a tube between the rod mover and the shaft to allow the rod to be inserted thereinto. The tube is inserted into a hole of the rod mover.

As described above, the surgical instrument according to the aspect of the present disclosure includes the tube between the rod mover and the shaft to allow the rod to be inserted thereinto. Accordingly, when the rod is pushed by the rod mover, buckling of the rod is reduced or prevented by the tube. Consequently, the bending angle of the bendable portion can be increased.

According to the present disclosure, it is possible to increase the bending angle of the bendable portion.

Brief Description of the Drawings

FIG. 1 is a diagram showing a surgical instrument according to a first embodiment, as viewed from a C1 direction side.
FIG. 2 is a diagram showing the surgical instrument according to the first embodiment, as viewed from a B2 direction side.
FIG. 3 is a diagram showing a shaft, a bendable portion, and an end effector according to the first embodiment.
FIG. 4 is a sectional view taken along the line 1300-1300 in FIG. 2.
FIG. 5 is a sectional view taken along the line 1000-1000 in FIG. 2.
FIG. 6 is a perspective view of the surgical instrument according to the first embodiment.
FIG. 7 is a sectional view taken along the line 1100-1100 in FIG. 2.
FIG. 8 is a sectional view taken along the line 1200-1200 in FIG. 2.
FIG. 9 is a diagram showing a distal end of a rod according to the first embodiment.
FIG. 10 is a sectional view taken along the line 1400-1400 in FIG. 2.
FIG. 11 is a diagram showing a movement mechanism according to the first embodiment.
FIG. 12 is a schematic view showing a rod mover, rods, tubes, and elastic members according to the first embodiment.
FIG. 13 is a diagram showing a state in which the bendable portion is bent to the B1 side.
FIG. 14 is a diagram showing a state in which the bendable portion is bent to the B2 side.
FIG. 15 is a schematic view showing a rod mover, rods, tubes, and elastic members according to a second embodiment.
FIG. 16 is a diagram showing a driver according to a modified example, as viewed from the C1 side.
FIG. 17 is a diagram for illustrating the operation of the driver according to the modified example.
FIG. 18 is a diagram showing the driver according to the modified example, as viewed from the B2 side.

Modes for Carrying Out the Invention

Embodiments embodying the present disclosure are hereinafter described on the basis of the drawings.

First Embodiment

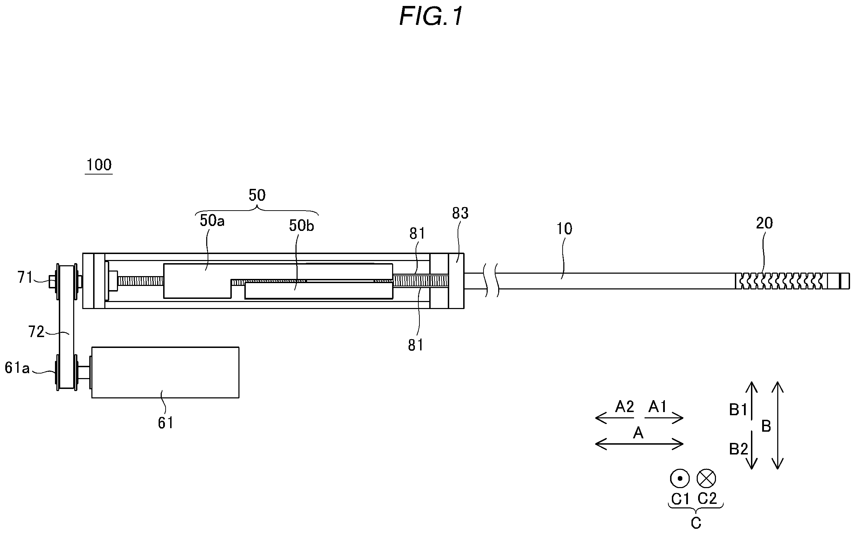

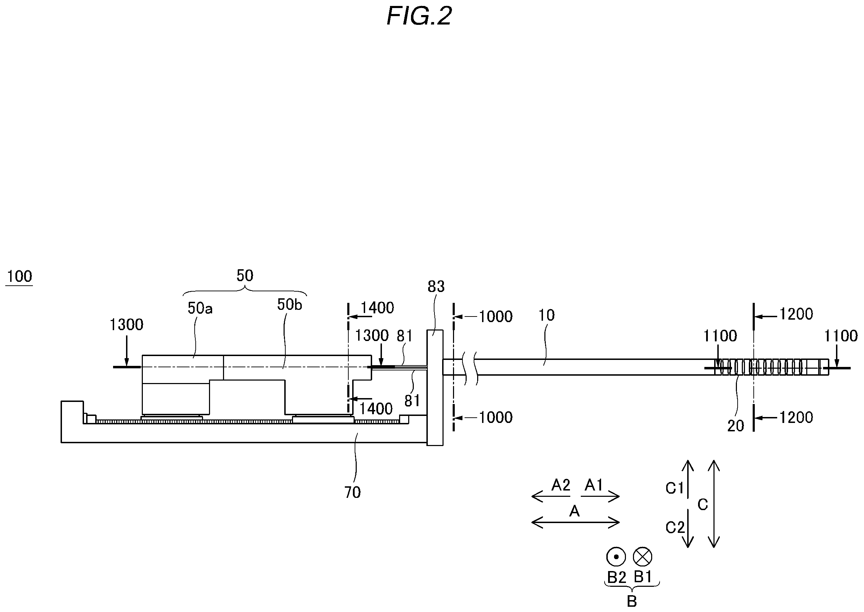

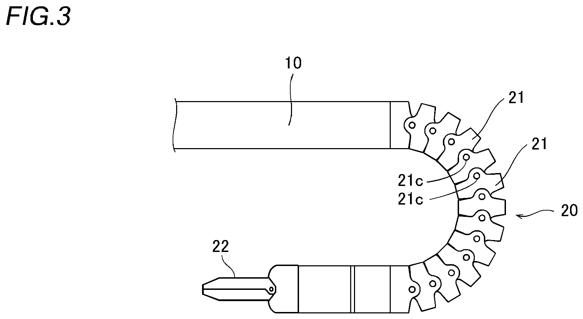

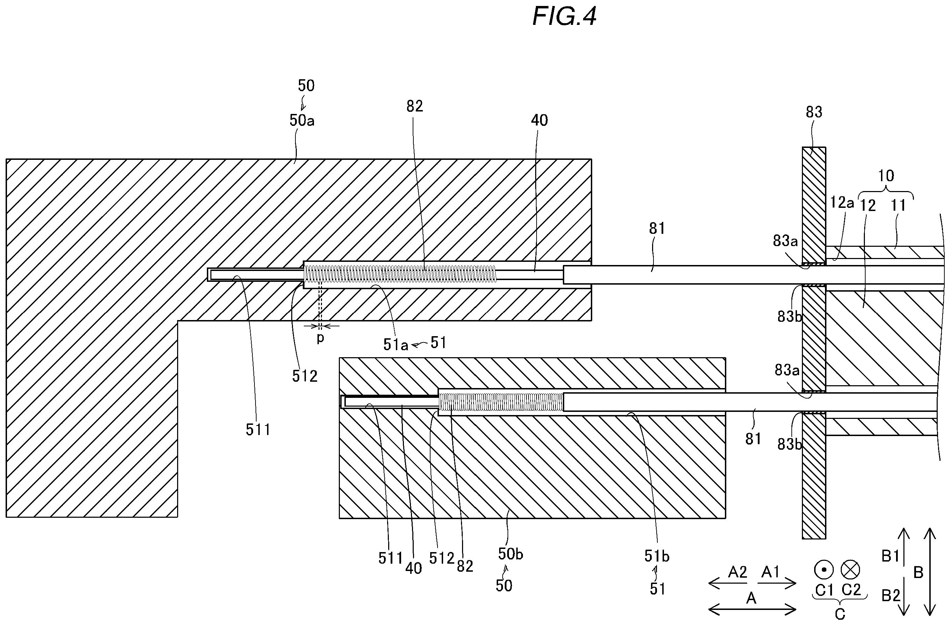

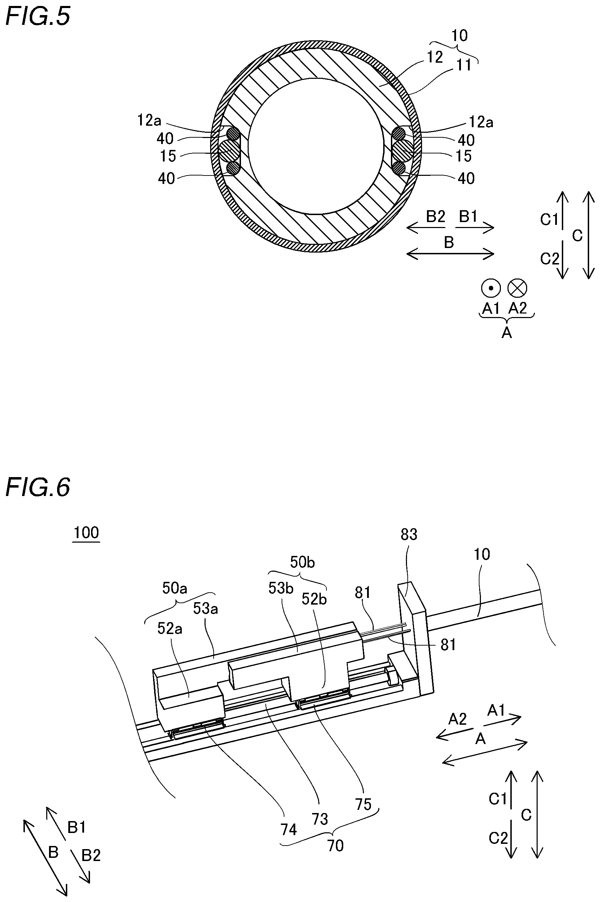

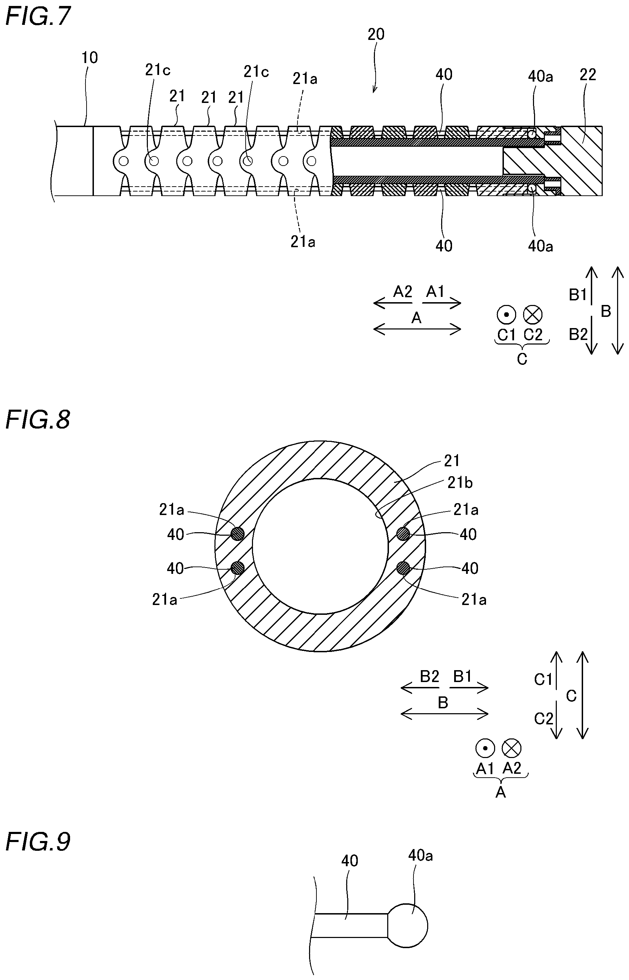

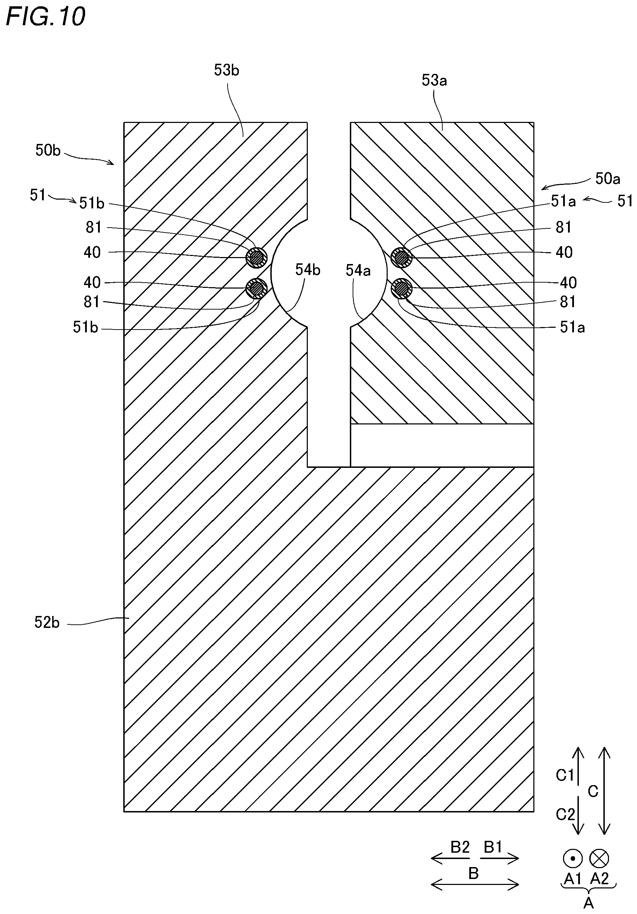

The configuration of a surgical instrument 100 according to a first embodiment is now described. In this description, the axial direction of a shaft 10 is defined as an A direction. The distal end side of the shaft 10 is defined as an A1 side, and the proximal end side of the shaft 10 is defined as an A2 side. A direction perpendicular to the axial direction of the shaft 10 is defined as a B direction. One side in the B direction is defined as a B1 side, and the other side in the B direction is defined as a B2 side. A direction perpendicular to the A direction and the B direction is defined as a C direction. One side in the C direction is defined as a C1 side, and the other side in the C direction is defined as a C2 side.
As shown in FIG. 1, the surgical instrument 100 includes the shaft 10, a bendable portion 20, a rod mover 50, a drive 61, and a fastener 83. As shown in FIG. 2, the surgical instrument 100 includes a driver 70. As shown in FIG. 3, the surgical instrument 100 includes an end effector 22. As shown in FIG. 4, the surgical instrument 100 includes rods 40, tubes 81, and elastic members 82. In FIG. 2, the drive 61 is omitted, for example. In addition, in the figures other than FIG. 3, the detailed structure of the end effector 22 is omitted. In FIG. 4, in order to make each component of the surgical instrument 100 easier to see, the width of the surgical instrument 100 in the A direction is reduced, and the width of the surgical instrument 100 in the B direction is expanded.
As shown in FIG. 1, the bendable portion 20 is connected to a distal end of the shaft 10. The rod mover 50 is disposed on the A2 side of the shaft 10, which is the proximal end side. The drive 61 is disposed on the B2 side of the rod mover 50. As shown in FIG. 2, the driver 70 is disposed on the C2 side of the rod mover 50. As shown in FIG. 3, the end effector 22 is connected to a distal end of the bendable portion 20. As shown in FIG. 4, the rods 40 are disposed inside the tubes 81 and the elastic members 82. The bendable portion 20 is bent by the rod mover 50 pushing and pulling the rods 40.
As shown in FIG. 5, the shaft 10 is a hollow tubular member. The shaft 10 includes an outer tube 11 and a grooved tube 12. The outer tube 11 covers the outer periphery of the grooved tube 12. Grooves 12a are formed in the grooved tube 12. The rods 40 are inserted into the grooves 12a. A pair of grooves 12a are formed. The grooves 12a are formed on the outer surface of the grooved tube 12 along the A direction, which is the axial direction of the shaft 10.
As shown in FIG. 7, the bendable portion 20 is connected to the distal end of the shaft 10. The bendable portion 20 includes a plurality of pieces 21. The plurality of pieces 21 are connected to each other. As shown in FIG. 8, each of the plurality of pieces 21 includes through-holes 21a and an internal space 21b. The rods 40 are inserted into the through-holes 21a. As shown in FIG. 7, the adjacent pieces 21 are coupled to each other by pins 21c. The distal end of the shaft 10 is coupled to the piece 21 located at a proximal end of the bendable portion 20 by the pin 21c. Each piece 21 is coupled to the adjacent piece 21 so as to be rotatable around the axis of the pin 21c. The axis of the pin 21c is an axis along the C direction. The axes of the pins 21c are parallel to each other. That is, the bendable portion 20 is bendable in an A-B plane perpendicular to the C direction.
As shown in FIG. 3, the end effector 22 is disposed at the distal end of the bendable portion 20. The end effector 22 is a pair of medical forceps, for example.
As shown in FIG. 7, the rods 40 are inserted into the shaft 10 and the bendable portion 20. Distal ends 40a of the rods 40 are fixed to the A1 side of the bendable portion 20, which is the end effector 22 side. The rods 40 are inserted into the grooves 12a provided in the grooved tube 12 and the through-holes 21a formed across the plurality of pieces 21. The distal ends 40a of the rods 40 engage with the piece 21 located at the distal end of the bendable portion 20. Specifically, as shown in FIG. 9, the distal end 40a of each of the rods 40 has a spherical shape. The rods 40 are inserted into the through-holes 21a from the distal end side of the bendable portion 20. Then, the spherical distal ends of the rods 40 engage with the through-holes 21a of the piece 21. Specifically, the spherical distal ends of the rods 40 push and pull the bendable portion 20. When the rods 40 push the bendable portion 20, the spherical distal ends of the rods 40 push the piece 21 that is located at the most distal end. When the rods 40 pull the bendable portion 20, the spherical distal ends of the rods 40 are hooked on the through-holes 21a of the piece 21, and the bendable portion 20 is pulled by the rods 40. The rods 40 are made of a rod-shaped superelastic alloy such as a nickel-titanium alloy. When the rods 40 are pushed and pulled, the bendable portion 20 bends in the A-B plane perpendicular to the C direction.
As shown in FIG. 5, two rods 40 are arranged in each groove 12a of the grooved tube 12 of the shaft 10. A spacer 15 is arranged between the two rods 40 arranged in one groove 12a. Buckling of the rods 40 in the groove 12a is prevented by the outer tube 11, the groove 12a of the grooved tube 12, and the spacer 15.
As shown in FIG. 4, the rod mover 50 has a block shape, for example. Holes 51 are formed in the rod mover 50. Proximal ends of the rods 40 are fixed to bottom portions 511 of the holes 51. The bottom portions 511 refer to portions of the holes 51 on the A2 side with respect to step portions 512. The proximal ends of the rods 40 are bonded, for example, by an adhesive applied to the entire bottom portions 511 of the holes 51. The rod mover 50 includes a pair of rod movers 50, a rod mover 50a and a rod mover 50b. Two rods 40 inserted into one of two grooves 12a of the grooved tube 12 are fixed to the rod mover 50a. Two rods 40 inserted into the other of the two grooves 12a are fixed to the rod mover 50b. In FIG. 4, one hole 51 is formed in the rod mover 50a and one rod 40 is inserted into the hole 51, but in reality, as shown in FIG. 10, two holes 51 are formed in the rod mover 50a, and two rods 40 are inserted into the holes 51, respectively. The same applies to the rod mover 50b. The holes 51 in the rod mover 50a are referred to as holes 51a. The holes 51 in the rod mover 50b are referred to as holes 51b. A recess 54a is formed in the rod mover 50a, and a recess 54b is formed in the rod mover 50b. A member to be inserted into the grooved tube 12 of the shaft 10 can be passed through the recess 54a and the recess 54b.

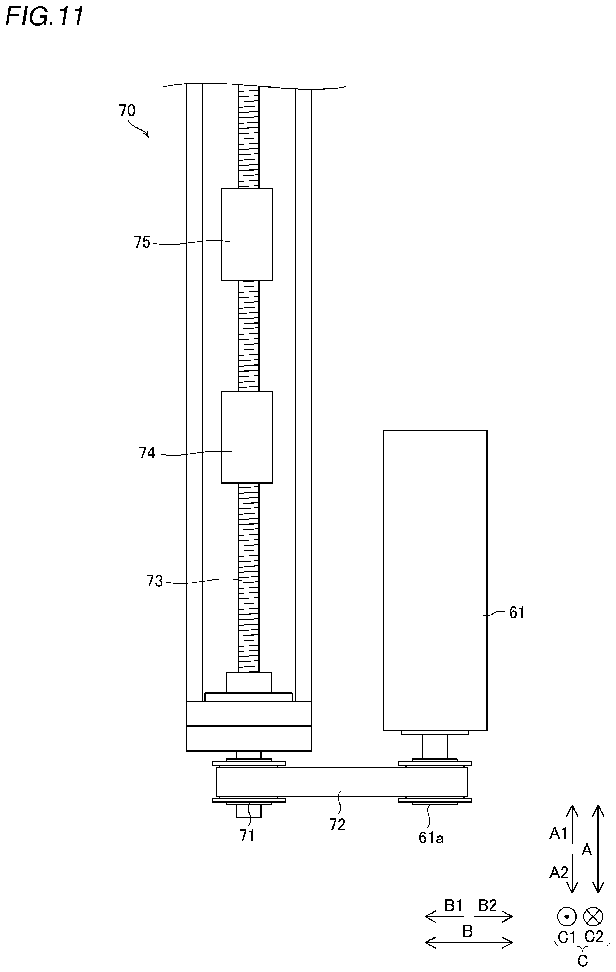

As shown in FIG. 11, the drive 61 reciprocates the rod mover 50 in the axial direction of the shaft 10. Specifically, in the first embodiment, the drive 61 includes one motor.

In the first embodiment, the driver 70 moves one of the pair of rod movers 50 in one direction in the A direction, which is the axial direction of the shaft 10, and moves the other of the pair of rod movers 50 in the other direction in the axial direction of the shaft 10, with the drive force of one motor. Specifically, the driver 70 includes a pulley 71, a timing belt 72, a ball screw 73, a movable body 74, and a movable body 75. A pulley 61a is connected to the drive 61. The timing belt 72 is arranged across the pulley 71 and the pulley 61a. The drive force of the drive 61 is transmitted to the ball screw 73 via the timing belt 72. The ball screw 73 includes a right-left screw. When the ball screw 73 rotates, the movable body 74 is moved to one side in the axial direction of the shaft 10, and the movable body 75 is moved to the other side in the axial direction of the shaft 10. The rod mover 50a and the rod mover 50b are attached to the movable body 74 and the movable body 75, respectively.

In the first embodiment, as shown in FIG. 6, the rod mover 50a includes a first portion 52a and a second portion 53a. The first portion 52a is connected to the driver 70. The second portion 53a is connected to the first portion 52a, and the rods 40 are connected to the second portion 53a. Similarly, the rod mover 50b includes a first portion 52b and a second portion 53b. The first portion 52a of the rod mover 50a and the first portion 52b of the rod mover 50b are arranged in series along the A direction, which is the axial direction of the shaft 10. The second portion 53a of the rod mover 50a is arranged on the B1 side, which is one side of the shaft 10, in the B direction perpendicular to the axial direction of the shaft 10. The second portion 53b of the rod mover 50b is arranged on the B2 side, which is one side of the shaft 10, in the B direction perpendicular to the axial direction of the shaft 10.

Specifically, the first portion 52a of the rod mover 50a is connected to the movable body 74 of the driver 70. The second portion 53a of the rod mover 50a extends in a C1 direction from the first portion 52a. The first portion 52b of the rod mover 50b is connected to the movable body 75 of the driver 70. The second portion 53b of the rod mover 50b extends in the C1 direction from the first portion 52b. Even when the rod mover 50a moves to the A1 side and the rod mover 50b moves to the A2 side due to rotation of the ball screw 73, the second portion 53a of the rod mover 50a and the second portion 53b of the rod mover 50b do not interfere with each other.

In the first embodiment, as shown in FIG. 4, the rods 40 are inserted into the tubes 81. The tubes 81 are disposed between the rod mover 50 and the shaft 10. The tubes 81 are inserted into the holes 51 of the rod mover 50. Specifically, each of the tubes 81 has a cylindrical shape. The inner and outer diameters of the tubes 81 are set such that the tubes 81 do not buckle together with the rods 40 when the rod mover 50 moves the rods 40 in an A1 direction. The tubes 81 are made of metal, for example. The proximal end sides of the tubes 81 are inserted into the holes 51 of the rod mover 50. Although FIG. 4 illustrates one tube 81 inserted into one hole 51a of the rod mover 50a, in reality, two holes 51a are formed in the rod mover 50a, two rods 40 are inserted into the holes 51a, respectively, and one tube 81 is disposed in each of the two holes 51a. The same applies to the rod mover 50b. That is, the tube 81 is disposed for each of four rods 40.

In the first embodiment, the fastener 83 fixes the A1 sides of the tubes 81, which are the shaft 10 sides. The A2 sides of the tubes 81, which are the rod mover 50 sides, are inserted into the holes 51 of the rod mover 50 and are free ends. The rod mover 50 is movable relative to the tubes 81. For example, the fastener 83 is a plate-shaped member arranged perpendicular to the A direction. Holes 83a are formed in the fastener 83. The rods 40 are inserted into the holes 83a. Ends of the tubes 81 on the A1 side are fixed to the holes 83a of the fastener 83. That is, the ends of the tubes 81 on the A1 side are fixed ends. The ends of the tubes 81 on the A1 side are fixed to the holes 83a of the fastener 83, for example, by an adhesive 83b. The ends of the tubes 81 on the A1 side may be fixed to the holes 83a of the fastener 83 by welding. Alternatively, male threads may be formed on the ends of the tubes 81 on the A1 side, and female threads may be formed in the holes 83a of the fastener 83, and the ends of the tubes 81 on the A1 side may be fixed to the holes 83a of the fastener 83 by screwing. Four holes 83a are formed in the fastener 83 corresponding to the four rods 40. Ends of the tubes 81 on the A2 side are not fixed to the holes 51 of the rod mover 50. Thus, even when the rod mover 50 moves to the A1 side or the A2 side, the tubes 81 do not move.

In the first embodiment, the elastic members 82 are disposed between the proximal ends of the rods 40 and the tubes 81 in the holes 51 of the rod mover 50, and cover the outer peripheries of the rods 40. The A1 sides of the elastic members 82 are free ends that are not fixed to the tubes 81. The A2 sides of the elastic members 82 are free ends that are not fixed. Although FIG. 4 illustrates one elastic member 82 disposed in one hole 51a of the rod mover 50a, in reality, two holes 51a are formed in the rod mover 50a, two rods 40 are inserted into the holes 51, respectively, and one elastic member 82 is disposed in each of the two holes 51. The same applies to the rod mover 50b.

In the first embodiment, each of the elastic members 82 includes a compression coil spring. The pitch p of the natural length of the compression coil spring is equal to or less than the buckling length l of the rod 40. The buckling length l is calculated by the following formula:

$$P_{cr} = n\pi^2 EI/l^2$$

where $P_{cr}$ is the buckling load, E is Young's modulus, I is the second moment of area, and n is the end modulus.

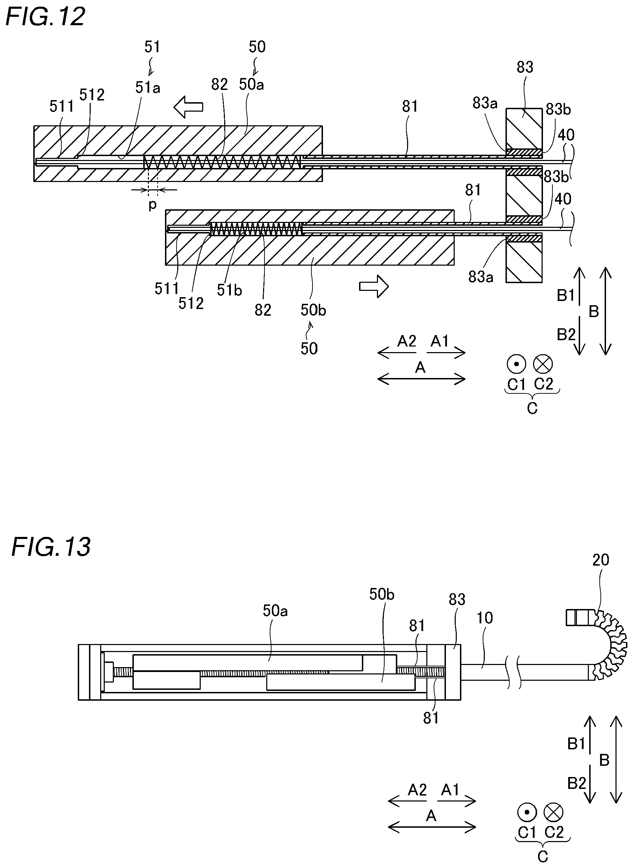

When the rod mover 50b moves to the A1 side as shown in FIG. 12, the elastic member 82 is compressed by the step portion 512 on the portion 511 side of the hole 51b of the rod mover 50b and the tube 81. Thus, buckling of the rod 40 is reduced or prevented by the tube 81 and the elastic member 82. When the rod mover 50a moves to the A2 side as shown in FIG. 12, the length of the elastic member 82 including a compression coil spring becomes its natural length.

Operation of Surgical Instrument

As shown in FIG. 13, the drive force of the drive 61 rotates the ball screw 73 of the driver 70 to one side such that the rod mover 50a is moved to the A2 side, and the rod mover 50b is moved to the A1 side. Thus, the bendable portion 20 is bent to the B1 side.

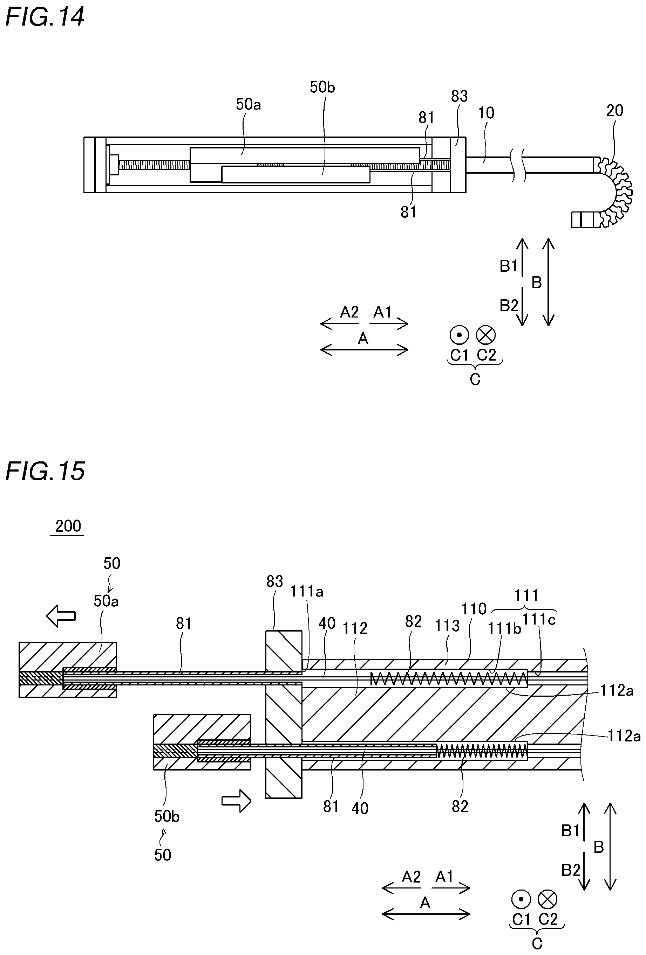

As shown in FIG. 14, the drive force of the drive 61 rotates the ball screw 73 of the driver 70 to the other side such that the rod mover 50a is moved to the A1 side, and the rod mover 50b is moved to the A2 side. Thus, the bendable portion 20 is bent to the B2 side.

Advantages of First Embodiment

The surgical instrument 100 includes the tubes 81 between the rod mover 50 and the shaft 10 to allow the rods 40 to be inserted thereinto. Accordingly, when the rods 40 are pushed by the rod mover 50, buckling of the rods 40 is reduced or prevented by the tubes 81. Consequently, the bending angle of the bendable portion 20 can be increased.

The portions of the rods 40 closer to the rod mover 50 are inserted into the holes 51 of the rod mover 50. The surgical instrument 100 includes the elastic members 82 between the proximal ends of the rods 40 and the tubes 81 in the holes 51 of the rod mover 50 to cover the outer peripheries of the rods 40. Accordingly, buckling of the rods 40 in the holes 51 of the rod mover 50 can be reduced or prevented by the elastic members 82.

Each of the elastic members 82 includes a compression coil spring, and the pitch p of the natural length of the compression coil spring is equal to or less than the buckling length l of the rod 40. Accordingly, buckling of the rod 40 in the hole 51 of the rod mover 50 can be reliably reduced or prevented by the compression coil spring.

A pair of rod movers 50 are arranged, and a plurality of rods 40 and a plurality of tubes 81 are arranged corresponding to the pair of rod movers 50. The drive 61 includes one motor. The surgical instrument 100 includes the driver 70 to move one of the pair of rod movers 50 to one side in the axial direction of the shaft 10 and move the other of the pair of rod movers 50 to the other side in the axial direction of the shaft 10 with the drive force of the single motor. Accordingly, the pair of rod movers 50 can be moved in opposite directions by the single motor, and thus the configuration of the surgical instrument 100 can be simplified and the motor can be easily controlled, as compared with a case in which a motor is arranged for each of the pair of rod movers 50.

The second portion 53a of the rod mover 50a is arranged on one side of the shaft 10 in the direction perpendicular to the axial direction of the shaft 10, and the second portion 53b of the rod mover 50b is arranged on the other side of the shaft 10 in the direction perpendicular to the axial direction of the shaft 10. Accordingly, the second portion 53a and the second portion 53b are arranged on different sides with respect to the shaft 10, and thus interference between the second portion 53a and the second portion 53b can be reduced or prevented.

Second Embodiment

A surgical instrument 200 according to a second embodiment is now described with reference to FIG. 15. In the surgical instrument 200, a shaft 110 includes a hole 111. The A1 side of a tube 81, which is the shaft 110 side, is inserted from an opening 111a of the hole 111 of the shaft 110 and is a free end. A rod mover 50 and the tube 81 are fixed, and the tube 81 moves inside the hole 111 of the shaft 110 together with the rod mover 50. An elastic member 82 is disposed on the A1 side of the tube 81, which is the shaft 110 side, inside the hole 111 of the shaft 110, and covers the outer periphery of a rod 40. Specifically, in the shaft 110, a groove 112a in which the elastic member 82 is disposed is formed in a portion on the A2 side of a grooved tube 112 of the shaft 110. The outer periphery of the grooved tube 112 is covered with an outer tube 113. The elastic member 82 is disposed in the hole 111 defined by a space surrounded by the groove 112a and the outer tube 113. The diameter of a portion 111b of the hole 111 on the A2 side in which the elastic member 82 is disposed is larger than the diameter of a portion 111c of the hole 111 on the A1 side. A proximal end of the tube 81 on the A2 side is fixed to the rod mover 50. When the rod mover 50 moves to the A1 side, the tube 81 is inserted into the hole 111, and the elastic member 82 contracts. When the rod mover 50 moves to the A2 side, the tube 81 slides inside the hole 111, and the elastic member 82 expands.

Advantages of Second Embodiment

The elastic member 82 covering the outer periphery of the rod 40 is arranged on the shaft 110 side of the tube 81 inside the hole 111 of the shaft 110, and thus buckling of the rod 40 inside the shaft 110 can be reduced or prevented by the elastic member 82. In addition, the elastic member 82 is arranged inside the shaft 110, and thus the rod mover 50 can be reduced in size as compared with a case in which the elastic member 82 is arranged inside the rod mover 50.

Modified Examples

The embodiments disclosed this time must be considered as illustrative in all points and not restrictive. The scope of the present disclosure is not shown by the above description of the embodiments but by the scope of claims for patent, and all modifications (modified examples) within the meaning and scope equivalent to the scope of claims for patent are further included.

While the example in which both the tube 81 and the elastic member 82 are provided to reduce or prevent buckling of the rod 40 has been shown in each of the first and second embodiments, the present disclosure is not limited to this. For example, only the tube 81 may be provided to reduce or prevent buckling of the rod 40.

While the example in which two rods 40 are connected to each of the rod mover 50a and the rod mover 50b has been shown in each of the first and second embodiments, the present disclosure is not limited to this. For example, only one rod 40 may be connected to each of the rod mover 50a and the rod mover 50b. Alternatively, three or more rods 40 may be connected to each of the rod mover 50a and the rod mover 50b.

While the example in which the elastic member 82 includes a compression coil spring has been shown in each of the first and second embodiments, the present disclosure is not limited to this. The elastic member 82 may include, for example, a soft tube that is expandable and contractable, other than a compression coil spring.

While the example in which both the rod mover 50a and the rod mover 50b are moved by one drive 61 has been shown in each of the first and second embodiments, the present disclosure is not limited to this. For example, a drive 61 may be arranged for each of the rod mover 50a and the rod mover 50b.

While the example in which the present disclosure is applied to the surgical instrument 100 has been shown in each of the first and second embodiments, the present disclosure is not limited to this. For example, the present disclosure may be applied to a mechanism including a bendable portion other than the surgical instrument 100.

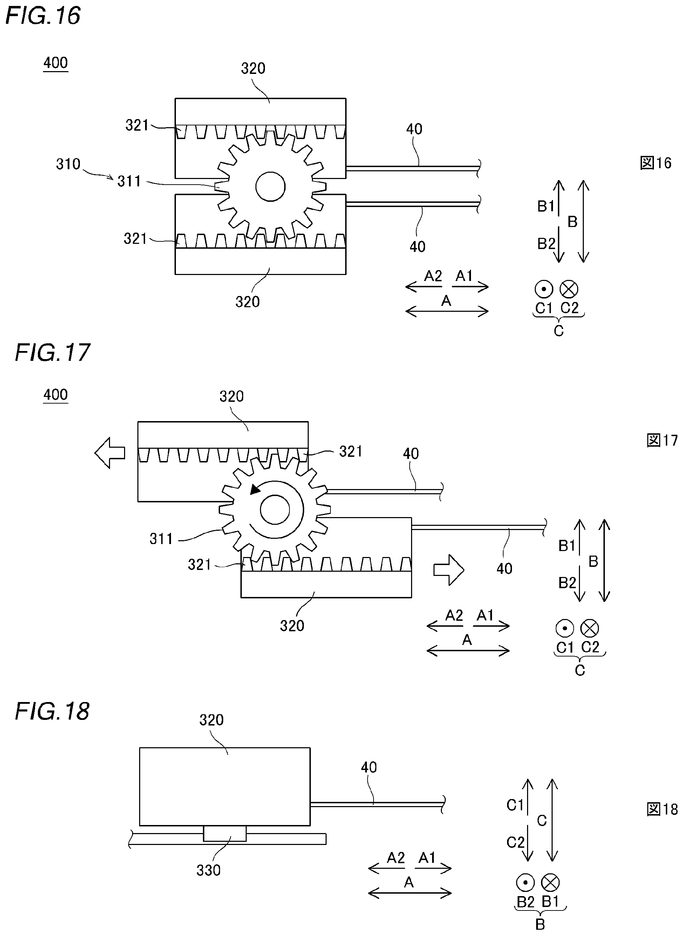

While the example in which the driver 70 includes the ball screw 73, the movable body 74, and the movable body 75 has been shown in each of the first and second embodiments, the present disclosure is not limited to this. For example, like a driver 310 of a surgical instrument 400 according to a modified example shown in FIG. 16, the driver 310 may include a pinion 311. A rack 321 is disposed on each of a pair of rod movers 320. As shown in FIG. 17, a drive (not shown) rotates the pinion 311 to move the pair of rod movers 320. Furthermore, as shown in FIG. 18, each of the pair of rod movers 320 is supported by a linear motion mechanism 330.

While the example in which the outer tube 11, the groove 12a of the grooved tube 12, and the spacer 15 prevent the rod 40 from buckling in the groove 12a has been shown in the first embodiment, the present disclosure is not limited to this. For example, the rod 40 disposed in the groove 12a of the grooved tube 12 may be surrounded by a guide pipe to be prevented from buckling in the groove 12a.

While the example in which both the A1 side and the A2 side of the elastic member 82 are free ends that are not fixed has been shown in the first embodiment, the present disclosure is not limited to this. For example, one of the A1 side and the A2 side of the elastic member 82 may be a free end, and the other may be a fixed end.

Aspects

It will be appreciated by those skilled in the art that the exemplary embodiments described above are specific examples of the following aspects.

(Item 1)

A surgical instrument comprising:

a shaft;
a bendable portion connected to a distal end of the shaft;
an end effector at a distal end of the bendable portion;
a rod inserted into the shaft and the bendable portion and including a distal end fixed to an end effector side of the bendable portion;
a rod mover to allow a proximal end of the rod to be fixed thereto;
a drive to reciprocate the rod mover in an axial direction of the shaft; and
a tube between the rod mover and the shaft to allow the rod to be inserted thereinto; wherein

the tube is inserted into a hole of the rod mover.

(Item 2)

The surgical instrument according to item 1, wherein

a portion of the rod closer to the rod mover is inserted into the hole of the rod mover; and
the surgical instrument further comprises an elastic member between the proximal end of the rod and the tube in the hole of the rod mover to cover an outer periphery of the rod.

(Item 3)

The surgical instrument according to item 2, wherein

the elastic member includes a compression coil spring; and
a pitch of a natural length of the compression coil spring is equal to or less than a buckling length of the rod.

(Item 4)

The surgical instrument according to item 3, further comprising:

a fastener to fix a shaft side of the tube; wherein
a rod mover side of the tube is inserted into the hole of the rod mover and is a free end; and
the rod mover is movable relative to the tube.

(Item 5)

The surgical instrument according to any one of items 1 to 3, wherein

the shaft includes a hole;
a shaft side of the tube is inserted from an opening of the hole of the shaft and is a free end movable relative to the opening of the shaft;
a rod mover side of the tube is fixed to the rod mover; and
the surgical instrument further comprises an elastic member on the shaft side of the tube inside the hole of the shaft to cover an outer periphery of the rod.

(Item 6)

The surgical instrument according to any one of items 1 to 5, wherein

the rod mover includes a pair of rod movers;
the rod and the tube include a plurality of rods and a plurality of tubes arranged corresponding to the pair of rod movers;
the drive includes one motor; and

the surgical instrument further comprises a driver to move one of the pair of rod movers to one side in the axial direction of the shaft and move the other of the pair of rod movers to the other side in the axial direction of the shaft with a drive force of the one motor.

(Item 7)

The surgical instrument according to item 6, wherein

each of the pair of rod movers includes:

a first portion connected to the driver; and
a second portion connected to the first portion and to allow the rod to be connected to the second portion;

the first portion of each of the pair of rod movers is arranged in series along the axial direction of the shaft;
the second portion of the one of the pair of rod movers is arranged on one side of the shaft in a direction perpendicular to the axial direction of the shaft; and
the second portion of the other of the pair of rod movers is arranged on the other side of the shaft in the direction perpendicular to the axial direction of the shaft.

**Claims**

1. A surgical instrument comprising:

a shaft;
a bendable portion connected to a distal end of the shaft;
an end effector at a distal end of the bendable portion;
a rod inserted into the shaft and the bendable portion and including a distal end fixed to an end effector side of the bendable portion;
a rod mover to allow a proximal end of the rod to be fixed thereto;
a drive to reciprocate the rod mover in an axial direction of the shaft; and
a tube between the rod mover and the shaft to allow the rod to be inserted thereinto; wherein
the tube is inserted into a hole of the rod mover.

2. The surgical instrument according to claim 1, wherein

a portion of the rod closer to the rod mover is inserted into the hole of the rod mover; and
the surgical instrument further comprises an elastic member between the proximal end of the rod and the tube in the hole of the rod mover to cover an outer periphery of the rod.

3. The surgical instrument according to claim 2, wherein

the elastic member includes a compression coil spring; and
a pitch of a natural length of the compression coil spring is equal to or less than a buckling length of the rod.

4. The surgical instrument according to claim 3, further comprising:

a fastener to fix a shaft side of the tube; wherein
a rod mover side of the tube is inserted into the hole of the rod mover and is a free end; and
the rod mover is movable relative to the tube.

5. The surgical instrument according to claim 1, wherein

the shaft includes a hole;
a shaft side of the tube is inserted from an opening of the hole of the shaft and is a free end movable relative to the opening of the hole of the shaft;
a rod mover side of the tube is fixed to the rod mover; and
the surgical instrument further comprises an elastic member on the shaft side of the tube inside the hole of the shaft to cover an outer periphery of the rod.

6. The surgical instrument according to claim 1, wherein

the rod mover includes a pair of rod movers;
the rod and the tube include a plurality of rods and a plurality of tubes arranged corresponding to the pair of rod movers;
the drive includes one motor; and
the surgical instrument further comprises a driver to move one of the pair of rod movers to one side in the axial direction of the shaft and move the other of the pair of rod movers to the other side in the axial direction of the shaft with a drive force of the one motor.

7. The surgical instrument according to claim 6, wherein

each of the pair of rod movers includes:

a first portion connected to the driver; and
a second portion connected to the first portion and to allow the rod to be connected to the second portion;

the first portion of each of the pair of rod movers is arranged in series along the axial direction of the shaft;
the second portion of the one of the pair of rod movers is arranged on one side of the shaft in a direction perpendicular to the axial direction of the shaft; and
the second portion of the other of the pair of rod movers is arranged on the other side of the shaft in the direction perpendicular to the axial direction of the shaft.

*FIG.1*

*FIG.2*

FIG.3

FIG.4

EP 4 613 216 A1

*FIG.5*

*FIG.6*

*FIG.7*

*FIG.8*

*FIG.9*

## FIG.10

*FIG.11*

**FIG.12**

**FIG.13**

FIG.14

FIG.15

FIG.16

400

320

321

310
311

40

40

321

320

B1
B2
B

A2 A1
A

⊙ ⊗
C1 C2
C

図16

FIG.17

400

320

321

40

311

40

321

320

B1
B2
B

A2 A1
A

⊙ ⊗
C1 C2
C

図17

FIG.18

320

40

330

C1
C2
C

A2 A1
A

⊙ ⊗
B2 B1
B

図18

## INTERNATIONAL SEARCH REPORT

| International application No. |
| --- |
| **PCT/JP2023/044577** |

### A. CLASSIFICATION OF SUBJECT MATTER

**_A61B 17/29_**(2006.01)i
FI:  A61B17/29

According to International Patent Classification (IPC) or to both national classification and IPC

### B. FIELDS SEARCHED

Minimum documentation searched (classification system followed by classification symbols)

A61B17/29

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2000-093522 A (CORDIS WEBSTER INC) 04 April 2000 (2000-04-04) | 1, 6, 7 |
|   | paragraphs [0006]-[0656], fig. 3, 11, 12 |   |
| A | paragraphs [0006]-[0656], fig. 3, 11, 12 | 2-5 |
| A | JP 2020-518353 A (CANON USA INC) 25 June 2020 (2020-06-25) | 1-7 |
|   | entire text, all drawings |   |
| A | JP 2009-297275 A (OLYMPUS CORPORATION) 24 December 2009 (2009-12-24) | 1-7 |
|   | entire text, all drawings |   |
| A | WO 2015/129438 A1 (OLYMPUS CORPORATION) 03 September 2015 (2015-09-03) | 1-7 |
|   | entire text, all drawings |   |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **24 January 2024** | **13 February 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 613 216 A1**

**INTERNATIONAL SEARCH REPORT**
Information on patent family members

| Patent document cited in search report | | | Publication date (day/month/year) | Patent family member(s) | | | Publication date (day/month/year) |
|---|---|---|---|---|---|---|---|
| JP | 2000-093522 | A | 04 April 2000 | US | 6120476 | A | |
| | | | | column 4, line 1 to column 14, line 37, fig. 3, 10, 11 | | | |
| | | | | EP | 928601 | A1 | |
| JP | 2020-518353 | A | 25 June 2020 | US | 2020/0383670 | A1 | |
| | | | | entire text, all drawings | | | |
| | | | | WO | 2018/204202 | A1 | |
| | | | | CN | 110621211 | A | |
| JP | 2009-297275 | A | 24 December 2009 | (Family: none) | | | |
| WO | 2015/129438 | A1 | 03 September 2015 | US | 2016/0360952 | A1 | |
| | | | | entire text, all drawings | | | |
| | | | | EP | 3111867 | A1 | |
| | | | | CN | 106061415 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014038075 A